# EUROPÄISCHE PATENTANMELDUNG

(11) **EP 0 539 920 A1**
(43) Veröffentlichungstag der Anmeldung: **05.05.1993**
(21) Anmeldenummer: 92118345.5
(22) Anmeldetag: 27.10.1992
(51) Int. Cl.: A61K 31/58, C07D 285/24

(54) **Arzneimittel zur Behandlung von AIDS**

(30) Priorität: 28.10.1991 DE 4135479
(71) Anmelder: GÖDECKE AKTIENGESELLSCHAFT, D-10587 Berlin (DE)
(72) Erfinder: Satzinger, Gerhard, Dr., W-7809 Denzlingen (DE)

(57) **Zusammenfassung**

Die Erfindung betrifft die Arzneimittel, enthaltend 3,4-Dihydro-1-phenyl-3-thio-1,2,4-benzothiadiazin-1-oxid, zur Protektion von Lymphozyten, besonders bei der Bekämpfung von AIDS.

## Beschreibung

AIDS (Acquired Immune Deficiency Syndrome) ist eine sehr ernst zu nehmende Infektionskrankheit, für die es bisher noch keine, zu einem vollständigen Heilungserfolg führende Therapie gibt.

Aus der DE-OS 25 30 792 ist 3,4-Dihydro-1-phenyl-3-thio-1,2,4-benzothiadiazin-1-oxid bekannt als Zwischenprodukt für die Synthese von Benzothiadiazinen mit hypotensiven, spasmolytischen, diuretischen und ZNS-dämpfenden Aktivitäten.

Es wurde nun überraschend gefunden, daß diese Verbindung Lymphozyten vollständig vor dem Angriff von HIV-Viren schützt und eine Hemmung der reversen Transkriptase bewirkt.

Gegenstand der vorliegenden Patentanmeldung sind daher Arzneimittel, enthaltend neben den üblichen Hilfs- und Zusatzstoffen 3,4-Dihydro-1-phenyl-3-thio-1,2,4-benzothiadiazin-1-oxid zur Protektion von Lymphozyten des Menschen und höherer Säugetiere, zur Hemmung der reversen Transkriptase und der Behandlung viraler Erkrankungen, insbesondere von AIDS.

Diese Verbindung stellt demnach ein Mittel dar, mit deren Hilfe durch Arzneimittel oder lymphozytotrope Viren bedingte toxische Wirkungen auf Lymphozyten verhindert werden können. Sie ermöglichen daher ein Therapiekonzept im Sinne einer selektiven Lymphozytenprotektion.

Unter lymphozytotoxischen Arzneimitteln werden im vorliegenden Zusammenhang Substanzen unterschiedlicher Wirkstoffklassen (z.B. Cytostatika, Antibiotika oder Antihypertensiva) verstanden, sofern diese entweder die Zahl der im Blut kreisenden Lymphozyten vermindern oder ihre immunologischen Funktionen beeinträchtigen. Beide Effekte führen zu einem mehr oder weniger ausgeprägten Versagen der Immunabwehr mit der Folge einer erhöhten Infektionsbereitschaft des Organismus. Bei dem erstgenannten Wirkungstyp, dem insbesondere lymphozytopenisch wirkende Cytostatika wie Cyclophosphamid zuzuordnen sind, kommt es aufgrund einer die Zellproliferation hemmenden Aktivität in den meisten Fällen zur Anhäufung unreifer und immuninkompetenter Lymphozyten-Vorstufen im Knochenmark, während gleichzeitig die Zahl der im Blut kreisenden reifen und immunologisch funktionsfähigen Lymphozyten abnimmt. Der zweite genannte Wirkungstyp führt bei unveränderter Lymphozytenzahl zu einer Veränderung von Lymphozytenfunktionen. Dieser Effekt kann auf sehr unterschiedliche Weise zustandekommen. Das Immunsuppressivum Cyclosporin A z.B. hemmt die Aktivierung der eigentlichen Abwehr-Lymphozyten (zytotoxische T-Lymphozyten) über eine Hemmung der Produktion des aktivierenden Botenstoffs durch die sogenannten T-Helferlymphozyten. Es wird bei dieser Substanz darüberhinaus auch eine Hemmung der Ausbildung jener Rezeptoren auf zytotoxischen T-Zellen diskutiert, an welche der sie aktivierende Botenstoff bindet.

Auch durch reversible Blockierung dieser Rezeptoren durch nichtzytotoxische Substanzen erzielt man auch einen Schutz der Lymphozytenoberfläche gegenüber lymphozytotropen Noxen.

Unter lymphozytotropen Noxen versteht man im vorliegenden Fall auch lymphozytotrope Viren. Bekanntlich geht der eigentlichen Infektion der Zelle durch diese Erreger zunächst deren Bindung an bestimmte Rezeptoren auf der Zelloberfläche voraus, wonach der Rezeptor-Virus-Komplex dann in das Zellinnere eingestülpt wird. Lymphozytotrope Viren können ihre Targetzellen entweder immortalisieren, d.h. sie maligne transformieren oder deren Elimination durch virusinduzierte Autoimmunprozesse (HIV im Fall von T-Helferzellen) provozieren. Für die genannten lymphozytotropen Virusinfektionen gibt es bisher keine wirksame Medikation. Die Patienten erliegen im allgemeinen aufgrund der virusinduzierten massiven Immuninsuffizienz generalisierten Infekten.

Die besondere Problematik der lymphozytotropen Viren im Vergleich zu den nicht lymphozytotropen Viren besteht demnach darin, daß sie bevorzugt Zellen des Immunsystems infizieren und daher genau die Mechanismen ausschalten, welche für die Elimination dieser Viren selbst wie auch begleitender opportunistischer Erreger unverzichtbar sind.

Aufgabe der Erfindung ist es, nicht zytotoxische und darüberhinaus durch eine hohe allgemeine Verträglichkeit gekennzeichnete Substanzen zu finden, welche für Lymphozyten toxische Bindungspartner chemischer oder viraler Art von ihren spezifischen Rezeptoren auf Lymphozytenoberflächen verdrängen oder ihre Interaktion mit diesen Targetzellen über andere Mechanismen behindern.

Im Fall der lymphozytotoxischen Cytostatika kann damit die bei diesen Antitumormitteln dosislimitierende und die Patienten häufig vital gefährdende Immunsuppression verhindert werden. Darüberhinaus kann man langfristig auch die bekannte Induktion von Sekundärtumoren durch Cytostatika beeinflussen, deren Entstehung durch die iatrogene Immunschwäche begünstigt wird.

Überraschenderweise wurde nun gefunden, daß 3,4-Dihydro-1-phenyl-3-thio-1,2,4-benzothiadiazin-1-oxid zu einem vollständigen Schutz von T-Lymphozyten gegenüber dem cytopathischen Effekt von HIV-Viren führt.

In Vergleichsversuchen wurden bei in-vitro Untersuchungen die folgenden Ergebnisse gefunden:
CPE-Zell-Linien-Assay gegen p24-Antigen, EC₅₀ ≈ 1,65 x 10⁻⁶;
CEM-IW-Zell-Linie, EC₅₀ ≈ 2 x 10⁻⁶;
Inhibierung der reversen Transkriptase, IC₅₀ = 2µM;
für die Zell-Linie C8166 wurden vergleichbare Werte gefunden, obwohl diese gewöhnlicherweise wesentlich schwieriger zu schützen ist, sodaß beispielsweise bei der Verwendung von AZT eine hundertfach höhere Dosis benötigt wird als bei Verwendung der erfindungsgemäßen Verbindung.

Im Fall lymphozytotroper Viren können erfindungsgemäß die zur Anheftung an Lymphozytenoberflächen von den Erregern benutzten Rezeptoren auf untoxische Weise blockiert werden. Hierbei ist an eine limitierte Prophylaxe in Risikosituationen zu denken, aber auch bei bereits manifestem Infekt wird aufgrund der Verhinderung der Infektion weiterer, vom Knochenmark nachgelieferter Immunzellen langfristig eine Restitution der Immunkompetenz erwartet. Es ist dann z.B. die bei HIV-Infekten (AIDS-Infektion) bisher nicht zu erzielende Erhaltung des Lebens der Patienten möglich.

3,4-Dihydro-1-phenyl-3-thio-1,2,4-benzothiadiazin-1-oxid kann erfindungsgemäß beim Menschen in flüssiger oder fester Form enteral oder parenteral appliziert werden. Als Injektionsmedium kommen vor allem wäßrige Phasen zur Anwendung, welche die üblichen Zusätze wie Stabilisierungsmittel und Lösungsvermittler enthalten. Derartige Zusätze sind z.B. Tartrat- und Citrat-Puffer, Ethanol, Komplexbildner (wie Äthylendiamin-tetraessigsäure und deren nicht-toxische Salze) sowie hochmolekulare Polymere (wie flüssiges Polyäthylenoxid) zur Viskositätsregulierung.

Feste Trägerstoffe sind z.B. Stärke, Lactose, Mannit, Methylcellulose, Talkum, hochdisperse Kieselsäuren, höhermolekulare Fettsäuren (wie Stearinsäure), Gelatine, Agar-Agar, Kalziumphosphat, Magnesiumstearat, tierische und pflanzliche Fette, feste hochmolekulare Polymere (wie Polyäthylenglykole); für orale Applikation geeignete Zubereitungen können gewünschtenfalls Geschmacks- und/oder Süßstoffe enthalten. Die enteral oder parenteral verabreichten Einzeldosen liegen im Bereich von 0,5 bis 1000 mg, vorzugsweise in Dosen von 1 bis 100 mg.

## Patentansprüche

1. Arzneimittel enthaltend 3,4-Dihydro-1-phenyl-3-thio-1,2,4-benzothiadiazin-1-oxid.

2. Verwendung von Arzneimitteln gemäß Anspruch 1 bei der Protektion von Lymphozyten des Menschen und höherer Säugetiere.

3. Verwendung von Arzneimitteln gemäß Anspruch 1 zur Inhibierung der reversen Transkriptase.

4. Verwendung von Arzneimitteln gemäß Anspruch 1 bei der Behandlung viraler Erkrankungen.

5. Verwendung von Arzneimitteln gemäß Anspruch 1 bei der Bekämpfung von AIDS.
